# EUROPEAN PATENT SPECIFICATION

(11) **EP 3 864 173 B1**
(45) Date of publication and mention of the grant of the patent: **27.08.2025**
(21) Application number: 19871480.0
(22) Date of filing: 09.10.2019
(51) Int. Cl.: C12Q 1/6806, C12Q 1/6869, C12Q 1/6837, C12Q 1/6841

(54) **SURFACE CAPTURE OF TARGET NUCLEIC ACID MOLECULES**
OBERFLÄCHENERFASSUNG VON ZIELNUKLEINSÄUREMOLEKÜLEN
CAPTURE DE SURFACE DES MOLECULES ACIDES DE NUCLEICQUES CIBLES

(30) Priority: 10.10.2018 US 201862743871 P; 08.07.2019 US 201962871421 P
(43) Date of publication of application: 18.08.2021
(73) Proprietor: 10x Genomics, Inc., Pleasanton, CA 94588-3260 (US)
(72) Inventor: DAUGHARTHY, Evan, Cambridge, Massachusetts 02139 (US); TERRY, Richard, Carlisle, Massachusetts 01741 (US)
(74) Representative: Grund, Martin
(86) International application number: PCT/US2019/055438
(87) International publication number: WO 2020/076979

(56) References cited:
- WO-A1-03/102233
- WO-A1-2012/140224
- WO-A2-2012/129242
- WO-A2-2017/019456
- US-A1- 2002 048 766
- US-A1- 2008 003 586
- US-A1- 2014 066 318
- US-A1- 2018 051 322
- US-A1- 2018 114 316

## Description

This application claims priority to U.S. Provisional Patent Application No. 62/743,871, filed October 10, 2018, and U.S. Provisional Patent Application No. 62/871,421, filed July 8, 2019.

### BACKGROUND

Transferring target molecules within a biological sample onto a planar surface can be useful for various downstream applications. For example, ribonucleic acid (RNA) molecules can be transferred to an array for transcriptomics detection. Diffusion may be used as the mechanism to transfer those target molecules to the planar surface.

WO2021/140224 discloses methods and products for the localized or spatial detection of nucleic acid in a tissue sample. US2018/114316 discloses methods and systems for preparing chromosomal spread for a selected cell so that chromosomal spreads and/or translocations can be correlated with the selected cell. US2018/051322 discloses methods of making a 3-D matrix of nucleic acids within a cell. US2002/048766 discloses methods of morphological reconstruction of biological activity in a tissue sample maps biological data resulting from analysis of tissue samples onto a 3-D morphological rendering of the biological sample. WO2017/019456 discloses methods and compositions for spatial detection and analysis of nucleic acids in a tissue sample.

### SUMMARY

The present invention provides a method for processing or analyzing a plurality of nucleic acid molecules in a biological sample, comprising:
(a) providing a biological sample to an array comprising a plurality of capture probes;
(b) using a pressure field directed through the biological sample to direct the plurality of nucleic acid molecules in the direction of the array comprising the plurality of capture probes;
(c) using at least a subset of the plurality of capture probes to capture at least a subset of the plurality of nucleic acid molecules, thereby immobilizing the at least the subset of the plurality of nucleic acid molecules to the array;
(d) identifying sequences and positions of the at least the subset of the plurality of nucleic acid molecules immobilized to the array; and
(e) using the positions identified in step (d) to identify the sequences as originating from corresponding positions in the biological sample;
wherein the biological sample is a cell or a tissue section.

Described herein are methods for processing or analyzing a plurality of nucleic acid molecules in a biological sample, comprising: (a) providing a biological sample adjacent to an array having a plurality of capture probes; (b) using a flow field directed through the biological sample to direct the plurality of nucleic acid molecules towards the array having the plurality of capture probes; (c) using at least a subset of the plurality of capture probes to capture at least a subset of the plurality of nucleic acid molecules, thereby immobilizing the at least the subset of the plurality of nucleic acid molecules adjacent to the array; (d) identifying sequences and positions of the at least the subset of the plurality of nucleic acid molecules immobilized adjacent to the array; and (e) using the positions identified in (d) to identify the sequences as having originated from positions within the biological sample.

In some embodiments, the flow field is an electric field. In some embodiments, the array is attached to a conductive solid substrate. In some embodiments, the electric field is generated by one or more anodes. In some embodiments, the one or more anodes are a spatial array of anodes. In some embodiments, each anode of the spatial array of anodes is co-localized with a subset of the plurality of the capture probes. In some embodiments, the one or more anodes are a continuous anode. In some embodiments, the flow field is a pressure field. In some embodiments, the pressure field is induced by positive or negative pressure. In some embodiments, the pressure field generates pressure-gradient forces. In some embodiments, the pressure field is an optical pressure field. In some embodiments, the optical pressure field is a radiation pressure field. In some embodiments, the optical pressure field is an optical gradient field. In some embodiments, the flow field is generated by radiation pressure. In some embodiments, the flow field is generated by optical gradient forces. In some embodiments, the flow field is spatially uniform across the biological sample. In some embodiments, the flow field is locally spatially uniform within one or more regions of the biological sample. In some embodiments, the flow field is locally spatially uniform within one or more regions of the biological sample, wherein the flow field directs the plurality of nucleic acid molecules of a local 3D volume of the biological sample to a subset of the plurality of capture probes.

In some embodiments, the biological sample is a cell or cell section. In some embodiments, the biological sample is fixed. In some embodiments, the biological sample is permeabilized. In some embodiments, the plurality of capture probes is immobilized to the array at individually addressable locations. In some embodiments, the plurality of capture probes is distributed in a spatially non-periodic manner. In some embodiments, the plurality of capture probes is distributed in a spatially periodic manner. In some embodiments, (d) comprises using detection probes to detect the sequences. In some embodiments, the plurality of capture probes is attached to a capture layer comprising a solid state, aqueous polymer or hydrogel layer. In some embodiments, (d) comprises subjecting the at least the subset of the plurality of nucleic acid molecules to sequencing. In some embodiments, the sequencing is performed using polymerase chain reaction (PCR).

In another aspect, the present disclosure describes a method for processing or analyzing a plurality of nucleic acid molecules in a biological sample, comprising: (a) providing a biological sample adjacent to an array having a plurality of capture probes under conditions sufficient to direct the plurality of nucleic acid molecules towards the array having the plurality of capture probes, wherein the plurality of nucleic acid molecules are towards the array at a rate that is greater than a rate of diffusion or gravity-assisted flow of the plurality of nucleic acid molecules in the biological sample; (b) using at least a subset of the plurality of capture probes to capture at least a subset of the plurality of nucleic acid molecules, thereby immobilizing the at least the subset of the plurality of nucleic acid molecules adjacent to the array; (c) identifying sequences and positions of the at least the subset of the plurality of nucleic acid molecules immobilized adjacent to the array; and (d) using the positions identified in (c) to identify the sequences as having originated from positions within the biological sample.

In some embodiments, the biological sample is a cell or cell section. In some embodiments, the biological sample is fixed. In some embodiments, the biological sample is permeabilized. In some embodiments, the plurality of capture probes are immobilized to the array at individually addressable locations. In some embodiments, the plurality of capture probes are distributed in a spatially non-periodic manner. In some embodiments, the plurality of capture probes are distributed in a spatially periodic manner. In some embodiments, (c) comprises using detection probes to detect the sequences. In some embodiments, the plurality of capture probes are attached to a capture layer comprising a solid state, aqueous polymer or hydrogel layer. In some embodiments, (c) comprises subjecting the at least the subset of the plurality of nucleic acid molecules to sequencing. In some embodiments, the sequencing is performed using polymerase chain reaction (PCR).

Another aspect of the present disclosure describes a non-transitory computer readable medium comprising machine executable code that, upon execution by one or more computer processors, implements any of the methods above or elsewhere herein.

Another aspect of the present disclosure describes a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described Accordingly, the drawings and description are to be regarded as illustrative in nature.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG. 1A** shows a schematic of an example embodiment of a method for analyzing nucleic acids.
**FIG. 1B** shows a schematic of an example embodiment of using an electric field to direct motion of nucleic acid molecules from a sample to a surface.
**FIG. 2** shows a computer system that is programmed or otherwise configured to implement methods provided herein.

### DETAILED DESCRIPTION

Various embodiments of the invention have been shown and described herein.

As used in the specification and claims, the singular form "a", "an" or "the" includes plural references unless the context clearly dictates otherwise. For example, the term "a cell" includes a plurality of cells, including mixtures thereof.

The term "nucleic acid," as used herein, generally refers to a nucleic acid molecule comprising a plurality of nucleotides or nucleotide analogs. A nucleic acid may be a polymeric form of nucleotides. A nucleic acid may comprise deoxyribonucleotides and/or ribonucleotides, or analogs thereof. A nucleic acid may be an oligonucleotide or a polynucleotide. Nucleic acids may have any three dimensional structure and may perform various functions. Non-limiting examples of nucleic acids include DNA, RNA, coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant nucleic acids, branched nucleic acids, plasmids, vectors, isolated DNA of any sequence, isolated RNA of any sequence, nucleic acid probes, and primers. A nucleic acid may comprise one or more modified nucleotides, such as methylated nucleotides and nucleotide analogs. If present, modifications to the nucleotide structure may be made before or after assembly of the nucleic acid. The sequence of nucleotides of a nucleic acid may be interrupted by non-nucleotide components. A nucleic acid may be further modified after polymerization, such as by conjugation, with a functional moiety for immobilization.

The term "capture probe," as used herein, generally refers to a molecule, such as a nucleic acid molecule (e.g., an oligonucleotide), that is configured to interact with a nucleic acid molecule, such as via hybridization (or other intermolecular interaction) or ligation. The capture probe can include a sequence that is complementary to a target sequence. For example, the capture probe can include a poly-T sequence (e.g., for capturing a messenger ribonucleic acid molecule) or a random N-mer. The capture probe can be configured to bind to a target or template nucleic acid molecule.

The capture probe may immobilize the nucleic acid molecule such that the nucleic acid molecule has fewer degrees of freedom. The capture probe may be attached to a substrate (e.g., an array or a bead), or otherwise constructed to be extracted or isolated from a solution. The substrate may be a solid or semi-solid substrate (e.g., a polymeric material). The capture probe may use a reaction to capture a nucleic acid molecule such that the nucleic acid becomes attached to the capture probe. For example nucleic acid that is proximal or adjacent to a capture probe may be attached via nucleic acid ligation. A capture probe may comprise a spatial index such that upon capture of a nucleic acid molecule, the spatial origin of a nucleic acid can be ascertained.

The term "spatial index," as used herein, generally refers to a probe, such as a nucleic acid molecule (e.g., an oligonucleotide), that is used to identify the spatial origin of another nucleic acid molecule (e.g., a target or template nucleic acid molecule, such as from a cell). A spatial index may comprise a specific sequence that can be indicative of particular location or spatial origin. The volume or area related to a spatial index may be as small as to encompass the space of a single molecule, or as large as to encompass multiple cells.

The term "detection probe," as used herein, generally refers to a probe, such as a nucleic acid molecule (e.g., oligonucleotide), that is used to detect another nucleic acid molecule (e.g., a target or template nucleic acid molecule, such as from a cell). The detection probe may hybridize to part or all of a nucleic acid sequence of a target nucleic acid molecule, for example. The detection probe may emit a signal (e.g., electromagnetic (or optical) signal or electrochemical signal) when the detection of a nucleic acid is performed.

Nucleic acids can be transferred to a surface for molecular indexing. For planar indexing schemes, the nucleic acids may be contacted with the planar substrate comprising the spatial indexing oligonucleotides. Existing methods may use diffusion, or the random motion of molecules, which can be self-propelled by thermal energy; the molecules can be captured by the planar indexing substrate, such as a microarray or polymer layer comprising the spatial index oligonucleotides; subsequently the spatial indices can be associated with the target nucleic acids, such as by polymerase extension or ligation; subsequently, both the spatial index and sequence of the target nucleic acid molecule can be determined, as by sequencing, enabling the user to infer the spatial origin of the target molecule within the sample.

However, the random motion of molecules may allow a molecule originating within the region of a certain spatial index to be captured by the spatial index of a separate region. This effect can be mitigated by methods of transporting the target nucleic acids to the planar capture surface using mechanisms for generating substantially directional, or substantially non-random, molecular motion perpendicular to the planar capture axis and in the direction of the capture plane.

Disclosed herein are methods, systems and compositions for processing or analyzing a plurality of nucleic acid molecules in a biological sample to identify a position of origin for at least a subset of the plurality of nucleic acid molecules. The methods and systems may comprise providing a sample which may be allowed to interact with the array of capture probes. In order to capture the nucleic acid molecules on the sample, a flow field may be used to direct the molecules of the sample to the capture probes. The nucleic acid molecules of the sample may then be immobilized or captured by the capture probe. Identification of the sequences and position of the captured nucleic molecules can be performed and the position on the array can be associated or mapped to a position in the sample. The nucleic acid sequence can then be determined to have originated from the mapped position of the sample.

Methods, systems, and compositions can be used for genomic, transcriptomic, proteomic, or other -omic analyses. Identifying an origin of a nucleic acid in a biological sample may for example, allow a determination of gene expression. Identifying an origin of a nucleic acid in a biological sample, may allow analysis of the presence of genetic aberrations, such as copy number variation, single nucleotide variation, deletions or insertion in a gene, or splice variants in RNA transcripts.

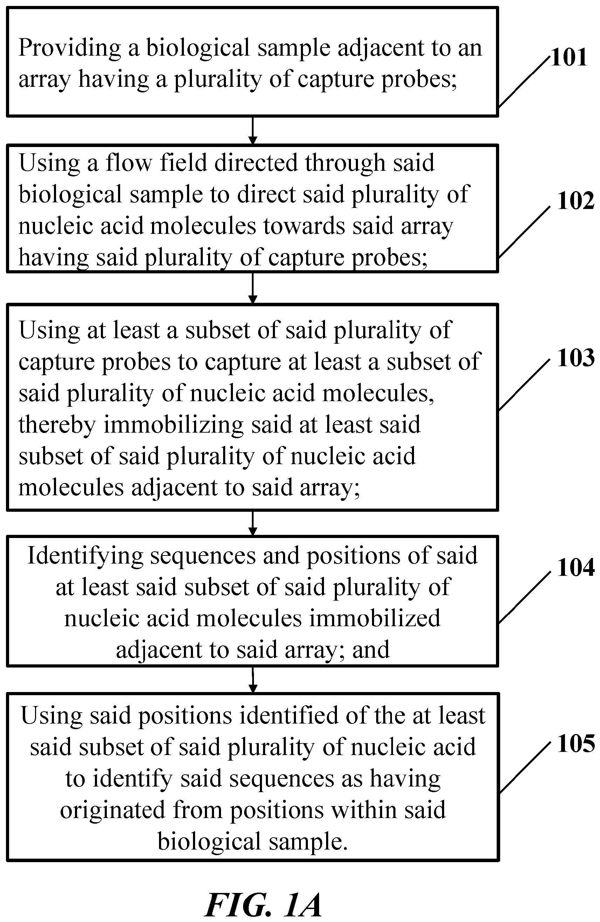

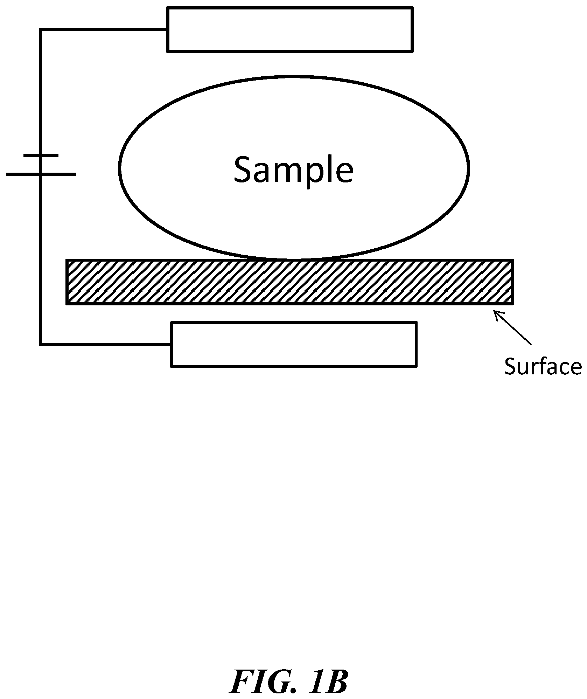

An example embodiment of a method is demonstrated by FIG. 1. In operation 101, a biological sample can be provided to an array of capture probes. Next, in operation 102, a flow field can then be used to direct nucleic acid molecules in the sample toward the capture probes. Next, in operation 103, a subset of molecules can then be captured by the probes and immobilized. Next, in operation 104, sequences and positions of the captured nucleic acids can then be identified. Next, in operation 105, the positions identified for the captured nucleic acids can then be used to identify the positions of origin for the nucleic acid sequences.

### Flow fields

To achieve a substantially directional molecular motion, a flow field may be used to transport the nucleic acids to the target nucleic acids to the index. The flow field may comprise an electric field. The flow field may comprise a pressure field or a pressure gradient. The flow field may be substantially uniform throughout the sample. The flow field may be non-uniform throughout the sample. The flow field may be constructed such that a first subset of nucleic acids is affected more than a second subset of nucleic acids. The flow field may be constructed such that a first subset of nucleic acids is affected equally as compared to a second subset of nucleic acids.

In some cases, electrical fields can be used to direct the motion of nucleic acid molecules. Due to the charged nature of nucleic acids under buffered conditions at a range of pH (e.g., under pH around neutral and basic pH), wherein nucleic acids bear a negative net charge, the net motion of nucleic acids may be directed using an electric field. The motion of particles relative to fluid under the influence of a spatially uniform electric field is known as electrophoresis. During electrophoresis, negatively charged nucleic acids can migrate toward a positively charged electrode (e.g., referred to as an anode).

Substantially directional electrophoretic transport of the target nucleic acids to the planar capture surface can be achieved by the generation of an electric field. According to one embodiment, the electric field can be spatially uniform or substantially spatially uniform, perpendicular to the planar capture surface, and with the anode-cathode-axis positioned with the anode in the direction of the planar capture surface. For example, the whole sample can be subjected to an electric field and the nucleic acid molecules can move perpendicularly to the planar capture surface. According to another embodiment, the electric field can be locally spatially uniform or substantially spatially uniform within one or more region(s) of the specimen and cognate planar capture surface. For example, the electric field can subject molecules in certain cell organelle and move those molecules to the planar capture surface, without capturing molecules in a different cell organelle on the planar capture surface. According to a separate aspect, the electric field exhibits one or more anodes such that the force of the electric field upon nucleic acids can attract nucleic acids within a certain 3D volume to a certain position or region of the planar capture substrate, wherein spatial indexing can occur. For example, the electric field can move the molecules in a larger 3D volume to a smaller spatial region on the planar capture surface. This may, for example, allow the molecules present in a cell organelle to be captured by a localized population of indices placed in a point in the middle of the cell organelle. In such an example, the localized population of indices may indicate that the spatial origin is that of a particular cell organelle.

Local directional capture of nucleic acids by electrokinetic transport can be enabled by the generation of one or more electric fields in relation to the capture substrate and biological specimen. In some cases, one or more anodes may be within the plane of spatial indexing. For example, the anode may be integrated into the planar capture surface. This may be done by integrating conductive material into the planar capture surface. The planar capture surface may be conductive or constructed of conductive material. The plane of spatial indexing may be between the anode and biological sample. For example the sample may be placed on top of the capture surface in which the capture surface is on top of an anode. One or more cathodes may be positioned distal to the planar capture surface relative to the biological specimen. For example, the arrangement may be that the biological sample is between one or more anodes and one or more cathodes. FIG. 1B shows an example arrangement of electrodes positioned on the top and the bottom of a sample (e.g., a cell sample or a tissue sample) and a surface for nucleic acids transfer. The surface can be placed in between positively charged electrode(s) and the sample such that nucleic acids of the sample can be directed by the electric field to migrate onto the surface.

Pressure fields or pressure-gradient forces may be used to transport nucleic acid molecules. The pressure field may be induced by positive or negative pressure. Pressure fields may be generated by air flow. For example, compressed air may be directed at the biological sample and/or the planar substrate. For example, the generation of a vacuum or suction can be used to direct molecules to the planar capture substrate. Pressure fields may comprise a radiation pressure field. For example, electromagnetic radiation may be used to apply to the biological sample to create a pressure field. The pressure field may comprise an optical pressure field or optical gradient field. For example, a beam of light may be used to generate pressure and move nucleic acid molecules to the capture substrate. One or more generators of light or other electromagnetic radiation may be used. Temperature gradients or heat generation may be used to generate pressure field. For example, a heat source may be used to create a temperature gradient which may generate a pressure differential. The generators of light or other electromagnetic radiation may be positioned as to generate spatial variation of flow fields as described elsewhere herein.

Flow field generators can be arranged or designed in various ways. For example, an electric field can be generated within the biological sample by one or more anode(s), a spatial array of anodes, or by a continuous anode, such that the electrokinetic motion of the nucleic acid molecules can be substantially towards and into and/or onto the capture layer. The flow field generators may be co-localized to spatial indices. For example, according to one embodiment of this aspect of the present disclosure, an array of anodes can be used such that the position of each anode is co-localized with a spatial index, such as by using an array of anode-indices, thereby forming an nucleic-acid attractive electric field within the vicinity of the sample proximal to the spatial index. An array of light sources may also be co-localized with spatial indices, as analogous to the anode-indices arrangement described above. Individual flow field generators can be operated independently of one another. For example, an individual anode in an anode array may be operated independently of another anode to allow an electric field to be generated in a local region on the sample.

Methods for planar capture can use a solid substrate, such as a glass slide or glass microarray slide as the planar capture substrate, either for spatial indexing by planar array or by region-of-interest capture. According to one embodiment of the present disclosure, the solid substrate can comprise an electrical circuit, comprising one or more anodes. Conductive materials may be integrated into, or printed onto, solid substrates such as glass and plastics, using various methods related to electrical engineering. According to another embodiment, the planar capture layer, wherein spatial indexing occurs, can be transparent or substantially conductive of the electric field. Further according to this embodiment, the planar capture layer can comprise a solid-state, aqueous polymer or hydrogel layer. The aqueous polymer or hydrogel layer may comprise polyacrylamide, poly(acrylate-co-acrylic acid) (PAA), Poly(N-isopropylacrylamide) (NIPAM), poly-ethylene-glycol (PEG) , or derivatives or combinations thereof. The aqueous polymer or hydrogel layer can be depolymerized or otherwise dissolved. This can allow the release of captured nucleic acids.

### Solid substrate

Solid substrates of the present disclosure may be fashioned into a variety of shapes. In certain embodiments, the solid substrate is substantially planar. Examples of solid substrates include plates such as slides, microtitre plates, flow cells, coverslips, microchips, and the like, containers such as microfuge tubes, test tubes and the like, tubing, sheets, pads, films and the like. Additionally, the solid substrates may be, for example, biological, nonbiological, organic, inorganic, or a combination thereof.

Solid substrate can be used interchangeably with solid surface or solid support and can include any material that can serve as a solid or semi-solid foundation for attachment of a biological sample other molecules such as polynucleotides, amplicons, DNA balls, other nucleic acids and/or other polymers, including biopolymers. Example types of materials comprising solid surfaces include, but are not limited to, glass, modified glass, functionalized glass, inorganic glasses, microspheres, including inert and/or magnetic particles, plastics, polysaccharides, nylon, nitrocellulose, ceramics, resins, silica, silica-based materials, carbon, metals, an optical fiber or optical fiber bundles, a variety of polymers other than those listed above and multiwell microtier plates. Example types of plastics include, but are not limited to, acrylics, polystyrene, copolymers of styrene and other materials, polypropylene, polyethylene, polybutylene, polyurethanes and Teflon^{™}. Example types of silica-based materials include, but are not limited to, silicon and various forms of modified silicon.

Solid substrates can also be varied in their shape depending on the application in a method described herein. For example, a solid substrate useful in the present disclosure can be planar, or contain regions which are concave or convex.

### Spatial indices

In methods disclosed herein, spatial indices are used to identify the spatial origin of nucleic acids. The spatial indices may be composed of nucleic acids, such as ribonucleic acids, deoxyribonucleic acids, or other nucleic acid derivatives. Spatial indices may have a known sequence or a randomly synthesized sequence. Spatial indices may be of a particular length. For example, the spatial index may be less than or equal to about 100, 90, 80, 70, 60, 50, 45, 40, 35, 30, 25, 20, 19, 18, 17, 16, 15, 14, 13, 12, 11, 10, 9, 8, 7, 6, 5, 4, 3, or 2 nucleotides long. For example, the spatial index may be greater than or equal to about 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, or more nucleotides long. Spatial indices may have no complementarity to the captured nucleic acids. Spatial indices may have a portion of its sequence have some complementarity with the captured nucleic acids as to hybridize or otherwise form a hydrogen bond with the captured nucleic acids. Spatial indices may be synthesized prior to coupling to the capture layer. Spatial indices may be synthesized *in situ* on the capture layer. The spatial indices may be synthesized using solid phase synthesis. Each spatial index in an array may have substantially the same sequence. A spatial index in an array may have a different sequence from another spatial index in an array.

According to this aspect of the present disclosure, a substantially 2-dimensional capture layer of spatial index nucleic acid molecules can be distributed, in either a known or random pattern. For example, the spatial index molecules may be arranged such the plurality of spatial indices or capture probes are distributed in a spatially periodic manner. For example, an array of indices may be a designed such that an index is present at every 1 nanometer. In another example, the plurality of capture probes may be distributed in a spatially non-periodic manner. For example, the indices may be allowed to disperse and then later attached to the capture layer. The dispersal of the indices may be done heterogeneously such that the resulting distribution is non-periodic. The spatial organization of the spatial indices and capture probes may be determined, such as by sequencing or detection by a detector probe. In some cases, the array of spatial indices is sparsely populated and there are few spatial indices for given volume. For example, in a given volume there may be more nucleic acid molecules than spatial indices. In some cases, the array of spatial indices is densely populated and there are many spatial indices for a given volume. For example, in a given volume there may be an equal amount of spatial indices as there are nucleic acid molecules. In another example, in a given volume there may be more spatial indices than there are nucleic acid molecules.

In methods disclosed herein, the spatial indices and/or capture probes may be attached to the capture layer. The spatial indices and/or capture probes may by covalently coupled to the capture layer. The spatial indices and/or capture probes may by non-covalently coupled to the capture layer. The spatial indices and/or capture probes may by adsorbed to a solid surface. The spatial indices and/or capture probes may use intermolecular forces to interact with the capture layer. The spatial indices and/or capture probes may use streptavidin and/or biotin to couple to the capture layer. The spatial indices and/or capture probes may be modified using chemical reactions such as alkylation, oxymercuration, periodate oxidation of RNA 3' vicinal diols, carbodiimide activation of RNA and DNA 5' phosphate, or by other nucleic-acid reactive chemistries such as psoralen and phenyl azide, for functional attachment of acryloyl or click-reactive moieties, which may be subsequently reacted with the capture later.

In methods disclosed herein, an array of spatial indices may interact with a biological sample. The array of spatial indices may interact with the whole area of a biological sample. The array of spatial indices may interact with a subset of the biological sample. The array of spatial indices may interact with a particular area of the biological sample. For example, the array of spatial indices may interact with nucleic acids from the nucleus of a cell, but not the mitochondria of the same cell.

Further according to this aspect of the present disclosure, the nucleic acids can be associated with the proximal spatial index, such as by primer extension, ligation, hybridization, and other methods related to nucleic acid biochemistry. For example, the capture probes may be specific to a particular gene or nucleic acid construct hybridize to a complementary sequence. The capture probe may be substantially non-specific to a particular gene. For example, the capture probe may comprise a poly-T portion, hybridize to a poly-A sequence and substantially capture mRNA. The capture probes may be ligated to the captured nucleic acid using a DNA ligase, RNA ligase, or non-specific nucleic acid ligase. For example a capture probe may be proximal to a nucleic acid molecule and a ligation reaction may occur to attach the capture probe to the nucleic acid molecule. Non limiting examples of ligation reactions include splint ligation, single-stranded DNA or RNA ligation, blunt-end ligation, cohesive-end ligation, hybrid DNA-RNA ligation, DNA-DNA ligation, RNA-RNA ligation, and circularization. An extension reaction can be performed to generate constructs of the nucleic acid attached to the capture probe and/or spatial index. For example, the capture probe may act as a primer and allow an extension reaction to occur generating substantially double stranded construct comprising the sequence of the capture probe and the captured nucleic acid molecule. In some cases, the capture probe and/or spatial index may act as part of the template of the extension reaction.

### Sample and sample preparation

Further according to this aspect of the present disclosure, a sample can be placed onto the capture layer. The sample may be a cell, a culture of cells, a cell section, or other cell derivative, a tissue, a tissue section, or other cell containing structures. The sample may be derived from a human. The sample may be derived from a non-human organism. The sample may be derived from a human with a disease or disorder. The sample may be derived from a diseased cell or tissue.

A sample can be a biological sample. A biological sample may be solid matter (e.g., biological tissue) or may be a fluid (e.g., a biological fluid). In general, a biological fluid can include any fluid associated with living organisms. Non-limiting examples of a biological sample include blood (or components of blood-e.g., white blood cells, red blood cells, platelets) obtained from any anatomical location (e.g., tissue, circulatory system, bone marrow) of a subject, cells obtained from any anatomical location of a subject, skin, heart, lung, kidney, breath, bone marrow, stool, semen, vaginal fluid, interstitial fluids derived from tumorous tissue, breast, pancreas, cerebral spinal fluid, tissue, throat swab, biopsy, placental fluid, amniotic fluid, liver, muscle, smooth muscle, bladder, gall bladder, colon, intestine, brain, cavity fluids, sputum, pus, micropiota, meconium, breast milk, prostate, esophagus, thyroid, serum, saliva, urine, gastric and digestive fluid, tears, ocular fluids, sweat, mucus, earwax, oil, glandular secretions, spinal fluid, hair, fingernails, skin cells, plasma, nasal swab or nasopharyngeal wash, spinal fluid, cord blood, emphatic fluids, and/or other excretions or body tissues. A biological sample may be a cell-free sample. Such cell-free sample may include DNA and/or RNA. A biological sample may be embedded in a matrix, e.g., a hydrogel matrix. The matrix may be a 3D matrix.

The sample, such as a culture of cells or tissue section, may be fixed, such as by the use of chemical fixatives or using various methods related to biological specimen fixation, such as to enable sectioning and/or partially or substantially stabilize the specimen during handling, deposition on the capture layer, and/or subsequent processing steps. For example, the sample may be fixed with formaldehyde, glutaraldehyde, ethanol, methanol, acetone, acetic acid, or a combination thereof. The fixation process may preserve nucleic acid molecules for subsequent capture and spatial indexing. The fixation process may selectively preserve nucleic acid molecules. The fixation process may, for example, remove or denature proteins or polypeptides. The fixation process may result in crosslinking of molecules in the biological sample. The sample may be frozen or embedded in wax to allow for sectioning. The sample may be immersed or soaked in a cryoprotectant or be flash frozen to prevent formation of ice crystals and better preserve the sample.

In some cases, the sample may be permeabilized, such as by using detergents and/or proteases or using other methods related to biological sample permeabilization, in order to enable the transport of target nucleic acid molecules to the capture layer. For example, saponin, Triton X-100, Tween-20, NP40, proteinase K, streptolysin O or a combination thereof may be used to permeabilize the sample. The detergents, proteases, or other permeabilizing agents may remove lipids and/or proteins from the sample. Removal of lipids or proteases may increase the overall efficiency of the subsequent capture and spatial indexing. For example, the sample may contain fewer molecules and thus the capture probe may have a higher probability of interacting with a nucleic acid molecule as opposed to a polypeptide or lipid molecule.

### Reactions of nucleic acids

In some cases, captured nucleic acids may be released. For example, the capture probes may be reversibly attached to a solid substrate, and this attachment may be reversed or cleaved to allow the capture probes to be released. In some case, the capture substrate may be dissolved allowing release of the nucleic acid. Upon release, the nucleic acids and/or capture probes may be isolated or collected and identified via method described elsewhere herein.

In some cases, nucleic acid molecules are subjected to amplification or extension reactions. For example, after capture of the nucleic acids, the captured nucleic acid molecule and spatial index can be subjected to an amplification or extension reaction. The resulting amplicon may have the sequence of the captured nucleic acid and the spatial index. In an example, amplicons corresponding to multiple captured nucleic acids/amplicons may be generated, collected, and pooled. The pooled amplicons can be subjected to sequencing reactions to identify nucleic acids and their respective spatial indices and thereby identify the identity and spatial origin on the nucleic acid molecules. The amplicons may be of a different type of nucleic acid as compared to the substrate sequence. For example, the amplification reaction may occur on RNA and result in cDNA via the enzymatic activity of a reverse transcriptase. Exemplary forms of nucleic acid amplification include Polymerase chain reaction (PCR), rolling circle amplification (RCA), loop mediated isothermal amplification (LAMP), nucleic acid sequence based amplification (NASBA), self-sustained sequence replication (3SR), strand displacement amplification, and multiple displacement amplification. Amplification may be mediated by an enzyme, for example, a polymerase. Non-limiting examples of polymerases include Phi29, Bst, Vent, 9°N, T4, Phusion DNA Polymerases, or T7, SP6 RNA polymerases.

Nucleic acid molecules and derivatives thereof may also be subjected to a number of reactions. For example, fragmentation, end-modification, second-stranding, annealing of accessory strands, such as priming, gap filling, circularization, blunt ending, phosphorylation, dephosphorylation, protection, and deprotection may be performed on nucleic acid molecules and derivatives thereof.

In various embodiments, nucleic acid molecules may be isolated. Isolation may be performed using nucleic acid isolation kits comprising nucleic acid binding columns, or other methods. Isolation may also be performed using phenol-chloroform extraction. The isolation techniques may otherwise remove contaminants such as proteins and lipids. The isolation may comprise isolating a particular type of nucleic acid. For example, DNase may be used in the isolation to remove DNA and effectively isolate RNA. In an alternate example, RNase may be used to remove RNA and effectively isolate DNA.

After capture and spatial indexing, the spatially indexed nucleic acids can be sequenced, determining both the spatial index and some part of the sequence of the target nucleic acid molecules. A sequence may be identified by nucleic acid amplification (e.g., polymerase chain reaction (PCR) or sequencing. Nucleic acid amplification may be performed by thermal cycling or under isothermal conditions (e.g., isothermal PCR). PCR may be digital PCR, real-time PCR (RTPCR), or quantitative PCR.

Sequencing may be performed using next generation sequencing platforms, for example, Illumina platforms, Pacific Biosciences of California, 454 Life Technology/Roche platforms, or SOLiD by Applied Biosystems. Sequencing may be whole genome sequencing, targeted sequenced, or random sequencing. Sequencing may be massively parallel array sequencing or single molecule sequencing. Sequencing may be performed using various sequencing techniques, such as, for example, sequencing by ligation, sequencing by synthesis, pyrosequencing, nanopore sequencing, polymerase chain reaction, or a combination thereof.

In some cases, detection of the sequences may comprise using a detection probe or a set of detection probes. For example, a detection probe may comprise sequences comprising a sequence or part of the sequence of a particular gene and/or spatial index. The detection probe may hybridize to a spatially indexed nucleic acid such that it is complementary to a portion of the spatial index and a portion of the captured nucleic acid molecule. The detection probe may have a reporter agent that may emit a signal when it is hybridized to a target molecule. For example, a detection probe may comprise a radioactive or fluorescent signal. The detection probe may emit a signal only when it interacts with the sequence that it detects. For example, the detect probe may be a molecular beacon probe. A detection probe may be used to detect the location of a particular spatial index in which had originally been allowed to randomly disperse.

In various embodiments, probes are used for capturing or detecting nucleic acids. The probes may be ribonucleic acid, deoxyribonucleic acid, or other derivatives or combinations thereof. The probes may be of a particular length. For example, the probes may be less than or equal to about 100, 90, 80, 70, 60, 50, 45, 40, 35, 30, 25, 20, 19, 18, 17, 16, 15, 14, 13, 12, 11, 10, 9, 8, 7, 6, 5, 4, 3, or 2 nucleotides long. For example, the probes may be greater than or equal to about 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, or more nucleotides long.

The methods disclosed herein may be performed at a particular temperature. The temperature may be uniform throughout the whole sample or substantially local to a specified area of the sample. Certain steps of a method may be performed at a particular temperature. For example, extension or amplification reactions may occur at 20 °C. Electrophoretic movement of the nucleic acid molecules, for example, may occur at temperature of 4 °C to reduce unwanted thermal motion of the nucleic acid molecules. In some cases, the temperature can affect the capture substrate. For example, hydrogels comprising NIPAM may be temperature sensitive and may increase or decrease in size.

The methods disclosed herein may be performed at a particular pH. The pH may be a basic, neutral or acidic pH. In the case of electrophoretic methods, the pH may be neutral or basic pH, such as a range from 6.0 - 10.0. In some cases, the pH is at least about 6.0, 6.5, 7.0, 7.5, 8.0, 8.5, 9.0, 9.5, or 10.0. A particular pH may be used or be optimized for a particular reaction described elsewhere herein. For example, ligation, extension, hybridization, isolation of molecules, may occur at a range of pHs.

### Computer control systems

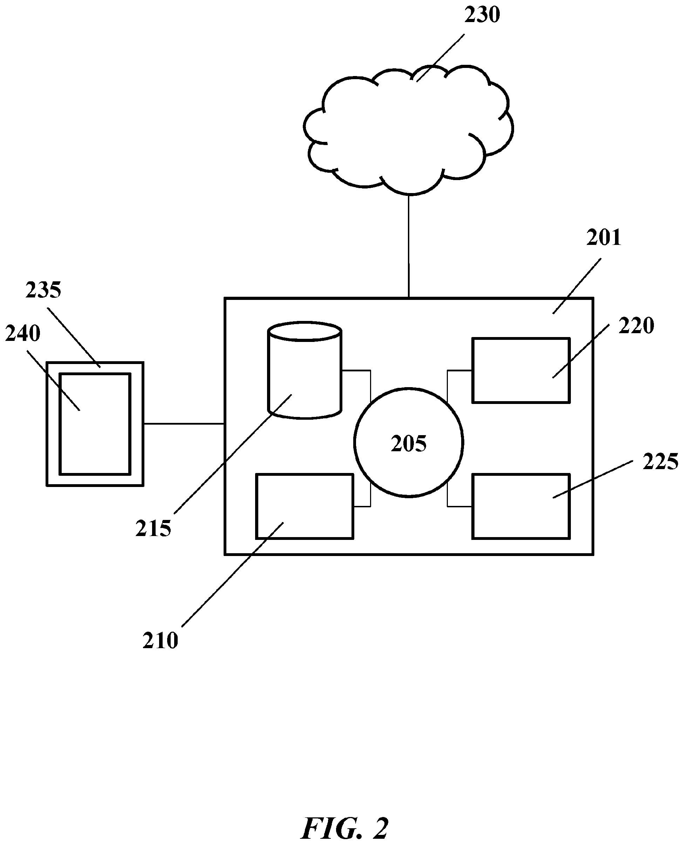

The present disclosure describes computer control systems that are programmed to implement methods of the disclosure. **FIG. 2** shows a computer system 201 that is programmed or otherwise configured to aid in generation of said libraries of probes, or sequencing nucleic acids of interest, as described here. The computer system 201 can regulate various aspects of the present disclosure, such as, for example, determination of target sequences of interest, and/or scoring of said probes. In some aspects, the computer system may be programmed to control release of reagents, activation of reactions (e.g., amplification reactions), and/or may initiate a sequencing reaction to take place. The computer system 201 can be an electronic device of a user or a computer system that is remotely located with respect to the electronic device. The electronic device can be a mobile electronic device.

The computer system 201 includes a central processing unit (CPU, also "processor" and "computer processor" herein) 205, which can be a single core or multi core processor, or a plurality of processors for parallel processing. The computer system 201 also includes memory or memory location 210 (e.g., random-access memory, read-only memory, flash memory), electronic storage unit 215 (e.g., hard disk), communication interface 220 (e.g., network adapter) for communicating with one or more other systems, and peripheral devices 225, such as cache, other memory, data storage and/or electronic display adapters. The memory 210, storage unit 215, interface 220 and peripheral devices 225 are in communication with the CPU 205 through a communication bus (solid lines), such as a motherboard. The storage unit 215 can be a data storage unit (or data repository) for storing data. The computer system 201 can be operatively coupled to a computer network ("network") 230 with the aid of the communication interface 220. The network 230 can be the Internet, an internet and/or extranet, or an intranet and/or extranet that is in communication with the Internet. The network 230 in some cases is a telecommunication and/or data network. The network 230 can include one or more computer servers, which can enable distributed computing, such as cloud computing. The network 230, in some cases with the aid of the computer system 201, can implement a peer-to-peer network, which may enable devices coupled to the computer system 201 to behave as a client or a server.

The CPU 205 can execute a sequence of machine-readable instructions, which can be embodied in a program or software. The instructions may be stored in a memory location, such as the memory 210. The instructions can be directed to the CPU 205, which can subsequently program or otherwise configure the CPU 205 to implement methods of the present disclosure. Examples of operations performed by the CPU 205 can include fetch, decode, execute, and writeback.

The CPU 205 can be part of a circuit, such as an integrated circuit. One or more other components of the system 201 can be included in the circuit. In some cases, the circuit is an application specific integrated circuit (ASIC).

The storage unit 215 can store files, such as drivers, libraries and saved programs. The storage unit 215 can store user data, e.g., user preferences and user programs. The computer system 201 in some cases can include one or more additional data storage units that are external to the computer system 201, such as located on a remote server that is in communication with the computer system 201 through an intranet or the Internet.

The computer system 201 can communicate with one or more remote computer systems through the network 230. For instance, the computer system 201 can communicate with a remote computer system of a user (e.g., a user generating said indices of the current disclosure or a user utilizing such indices). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 201 via the network 230.

Methods as described herein can be implemented by way of machine (e.g., computer processor) executable code stored on an electronic storage location of the computer system 201, such as, for example, on the memory 210 or electronic storage unit 215. The machine executable or machine readable code can be provided in the form of software. During use, the code can be executed by the processor 205. In some cases, the code can be retrieved from the storage unit 215 and stored on the memory 210 for ready access by the processor 205. In some situations, the electronic storage unit 215 can be precluded, and machine-executable instructions are stored on memory 210.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods described herein, such as the computer system 201, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 201 can include or be in communication with an electronic display 235 that comprises a user interface (UI) 240 for providing, for example, spatial origin of nucleic acid molecules, or showing detection and/or sequencing of biomolecules of interest. Examples of UI's include, without limitation, a graphical user interface (GUI) and web-based user interface. In some instances, the computer system may be configured to be in communication with various other devices and may be programmed to control such devices. For example, the computer system may be in communication with various light sources (e.g., fluorescent light sources) and/or platforms for utilizing said probe libraries or platforms utilized for sequencing.

Methods and systems of the present disclosure can be implemented by way of one or more algorithms. An algorithm can be implemented by way of software upon execution by the central processing unit 205. The algorithm can, for example, be executed so as to generate said indices of the current disclosure. The algorithms may comprise relevant parameters for designing and/or generating said probes. In some instances, the algorithms may comprise relevant parameters to implement detection of biomolecules of interest.

Several aspects are described with reference to example applications for illustration. Unless otherwise indicated, any embodiment may be combined with any other embodiment. It should be understood that numerous specific details, relationships, and methods are set forth to provide a full understanding of the features described herein. A skilled artisan, however, will readily recognize that the features described herein may be practiced without one or more of the specific details or with other methods. The features described herein are not limited by the illustrated ordering of acts or events, as some acts may occur in different orders and/or concurrently with other acts or events. Furthermore, not all illustrated acts or events are required to implement a methodology in accordance with the features described herein.

Some embodiments described herein contemplate numerical ranges. When ranges are present, the ranges include the range endpoints. Additionally, every sub range and value within the range is present as if explicitly written out. The term "about" or "approximately," unless otherwise stated, generally means within an acceptable error range for the particular value, which may depend at least in part on how the value is measured or determined, e.g., the limitations of the measurement system. For example, "about" may mean within 1 or more than 1 standard deviation, per the practice in the art. Alternatively, "about" may mean a range of up to 20%, up to 10%, up to 5%, or up to 1% of a given value. Alternatively, particularly with respect to biological systems or processes, the term may mean within an order of magnitude, within 5-fold, or within 2-fold, of a value.

## Claims

1. A method for processing or analyzing a plurality of nucleic acid molecules in a biological sample, comprising:
(a) providing a biological sample to an array comprising a plurality of capture probes;
(b) using a pressure field directed through the biological sample to direct the plurality of nucleic acid molecules in the direction of the array comprising the plurality of capture probes;
(c) using at least a subset of the plurality of capture probes to capture at least a subset of the plurality of nucleic acid molecules, thereby immobilizing the at least the subset of the plurality of nucleic acid molecules to the array;
(d) identifying sequences and positions of the at least the subset of the plurality of nucleic acid molecules immobilized to the array; and
(e) using the positions identified in step (d) to identify the sequences as originating from corresponding positions in the biological sample;
wherein the biological sample is a cell or a tissue section.

2. The method of claim 1, wherein the pressure field is induced by positive or negative pressure, or generates pressure-gradient forces.

3. The method of claim 1, wherein the pressure field is an optical pressure field, e.g., a radiation pressure field or an optical gradient field.

4. The method of claim 1, wherein the pressure field is spatially uniform across the biological sample.

5. The method of claim 1, wherein the pressure field is locally spatially uniform within one or more regions of the biological sample.

6. The method of claim 1, wherein the pressure field is locally spatially uniform within one or more regions of the biological sample, wherein the pressure field directs the plurality of nucleic acid molecules of a local 3D volume of the biological sample to a subset of the plurality of capture probes.

7. The method of claim 1, wherein the tissue section is a fixed tissue section.

8. The method of claim 1, wherein the biological sample is permeabilized.

9. The method of claim 1, wherein the plurality of capture probes are immobilized to the array at individually addressable locations.

10. The method of claim 1, wherein the plurality of capture probes are distributed in a spatially non-periodic manner.

11. The method of claim 1, wherein the plurality of capture probes are distributed in a spatially periodic manner.

12. The method of claim 1, wherein step (d) comprises using one or more detection probe(s) to identify the sequences.

13. The method of claim 1, wherein the plurality of capture probes are attached to a capture layer comprising a solid state, aqueous polymer, or hydrogel.

14. The method of claim 1, wherein the identifying in step (d) comprises sequencing the at least the subset of the plurality of nucleic acid molecules.

## Patentansprüche

1. Verfahren für ein Verarbeiten oder Analysieren einer Mehrzahl von Nukleinsäuremolekülen in einer biologischen Probe, umfassend:
(a) Bereitstellen einer biologischen Probe auf einer Anordnung, die eine Mehrzahl von Erfassungssonden umfasst;
(b) Verwenden eines durch die biologische Probe geleiteten Druckfeldes, um die Mehrzahl von Nukleinsäuremolekülen in die Richtung der Anordnung zu leiten, die die Mehrzahl von Erfassungssonden umfasst;
(c) Verwenden mindestens einer Teilmenge der Mehrzahl von Erfassungssonden, um mindestens eine Teilmenge der Mehrzahl von Nukleinsäuremolekülen zu erfassen, wodurch die mindestens eine Teilmenge der Mehrzahl von Nukleinsäuremolekülen auf der Anordnung immobilisiert wird;
(d) Identifizieren von Sequenzen und Positionen von mindestens der Teilmenge der Mehrzahl von Nukleinsäuremolekülen, die auf der Anordnung immobilisiert sind; und
(e) Verwenden der in Schritt (d) identifizierten Positionen, um die Sequenzen als aus entsprechenden Positionen in der biologischen Probe stammend zu identifizieren;
wobei die biologische Probe eine Zelle oder ein Gewebeschnitt ist.

2. Verfahren nach Anspruch 1, wobei das Druckfeld durch Überdruck oder Unterdruck induziert wird oder Druckgradientenkräfte erzeugt.

3. Verfahren nach Anspruch 1, wobei das Druckfeld ein optisches Druckfeld ist, z. B. ein Strahlungsdruckfeld oder ein optisches Gradientenfeld.

4. Verfahren nach Anspruch 1, wobei das Druckfeld über die biologische Probe räumlich gleichmäßig ist.

5. Verfahren nach Anspruch 1, wobei das Druckfeld innerhalb einer oder mehrerer Regionen der biologischen Probe lokal räumlich gleichmäßig ist.

6. Verfahren nach Anspruch 1, wobei das Druckfeld innerhalb einer oder mehrerer Regionen der biologischen Probe lokal räumlich gleichmäßig ist, wobei das Druckfeld die Mehrzahl von Nukleinsäuremolekülen eines lokalen 3D-Volumens der biologischen Probe zu einer Teilmenge der Mehrzahl von Erfassungssonden leitet.

7. Verfahren nach Anspruch 1, wobei der Gewebeschnitt ein fixierter Gewebeschnitt ist.

8. Verfahren nach Anspruch 1, wobei die biologische Probe permeabilisiert wird.

9. Verfahren nach Anspruch 1, wobei die Mehrzahl von Erfassungssonden an individuell adressierbaren Stellen auf der Anordnung immobilisiert sind.

10. Verfahren nach Anspruch 1, wobei die Mehrzahl von Erfassungssonden in einer räumlich nicht periodischen Weise verteilt sind.

11. Verfahren nach Anspruch 1, wobei die Mehrzahl von Erfassungssonden in einer räumlich periodischen Weise verteilt ist.

12. Verfahren nach Anspruch 1, wobei Schritt (d) ein Verwenden einer oder mehrerer Nachweissonde(n) umfasst, um die Sequenzen zu identifizieren.

13. Verfahren nach Anspruch 1, wobei die Mehrzahl von Erfassungssonden an einer Erfassungsschicht angelagert ist, die einen Festkörper, ein wässriges Polymer oder ein Hydrogel umfasst.

14. Verfahren nach Anspruch 1, wobei das Identifizieren in Schritt (d) ein Sequenzieren mindestens der Teilmenge der Mehrzahl von Nukleinsäuremolekülen umfasst.

## Revendications

1. Procédé pour traiter ou analyser une pluralité de molécules d'acide nucléique dans un échantillon biologique, comprenant :
(a) la fourniture d'un échantillon biologique à un réseau comprenant une pluralité de sondes de capture ;
(b) l'utilisation d'un champ de pression dirigé à travers l'échantillon biologique pour diriger la pluralité de molécules d'acide nucléique dans la direction du réseau comprenant la pluralité de sondes de capture ;
(c) l'utilisation de l'au moins un sous-ensemble de la pluralité de sondes de capture pour capturer l'au moins un sous-ensemble de la pluralité de molécules d'acide nucléique, immobilisant ainsi l'au moins le sous-ensemble de la pluralité de molécules d'acide nucléique sur le réseau ;
(d) l'identification de séquences et de positions de l'au moins le sous-ensemble de la pluralité de molécules d'acide nucléique immobilisées sur le réseau ; et
(e) l'utilisation des positions identifiées à l'étape (d) pour identifier les séquences comme provenant de positions correspondantes dans l'échantillon biologique ;
dans lequel l'échantillon biologique est une cellule ou une section de tissu.

2. Procédé selon la revendication 1, dans lequel le champ de pression est induit par une pression positive ou négative, ou génère des forces de pression.

3. Procédé selon la revendication 1, dans lequel le champ de pression est un champ de pression optique, par exemple un champ de pression de radiation ou un champ de gradient optique.

4. Procédé selon la revendication 1, dans lequel le champ de pression est spatialement uniforme sur l'échantillon biologique.

5. Procédé selon la revendication 1, dans lequel le champ de pression est localement spatialement uniforme au sein d'une ou plusieurs régions de l'échantillon biologique.

6. Procédé selon la revendication 1, dans lequel le champ de pression est localement spatialement uniforme au sein d'une ou plusieurs régions de l'échantillon biologique, dans lequel le champ de pression dirige la pluralité de molécules d'acide nucléique d'un volume 3D local de l'échantillon biologique vers un sous-ensemble de la pluralité de sondes de capture.

7. Procédé selon la revendication 1, dans lequel la section de tissu est une section de tissu fixé.

8. Procédé selon la revendication 1, dans lequel l'échantillon biologique est perméabilisé.

9. Procédé selon la revendication 1, dans lequel la pluralité de sondes de capture sont immobilisées sur le réseau à des emplacements adressables individuellement.

10. Procédé selon la revendication 1, dans lequel la pluralité de sondes de capture sont distribuées d'une manière spatialement non périodique.

11. Procédé selon la revendication 1, dans lequel la pluralité de sondes de capture sont distribuées d'une manière spatialement périodique.

12. Procédé selon la revendication 1, dans lequel l'étape (d) comprend l'utilisation d'une ou plusieurs sonde(s) de détection pour identifier les séquences.

13. Procédé selon la revendication 1, dans lequel la pluralité de sondes de capture sont fixées à une couche de capture comprenant un état solide, un polymère aqueux ou un hydrogel.

14. Procédé selon la revendication 1, dans lequel l'identification dans l'étape (d) comprend le séquençage de l'au moins le sous-ensemble de la pluralité de molécules d'acide nucléique.
